(19) Europäisches Patentamt
European Patent Office
Office européen des brevets

(11) Publication number: **0 028 083**
**B1**

(12) # EUROPEAN PATENT SPECIFICATION

(45) Date of publication of patent specification: **08.02.84**

(21) Application number: **80303552.6**

(22) Date of filing: **09.10.80**

(51) Int. Cl.³: **C 07 D 498/04,**
A 61 K 31/42 //(C07D498/04, 263/00, 205/00)

(54) **Derivatives of clavulanic acid, a process for their preparation and pharmaceutical compositions containing them.**

(30) Priority: **26.10.79 GB 7937261**

(43) Date of publication of application:
**06.05.81 Bulletin 81/18**

(45) Publication of the grant of the patent:
**08.02.84 Bulletin 84/6**

(84) Designated Contracting States:
**CH DE FR GB IT LI NL**

(56) References cited:
EP - A - 0 008 877
DE - A - 2 646 003
DE - A - 2 725 203
GB - A - 2 007 667

(73) Proprietor: **BEECHAM GROUP PLC**
**Beecham House Great West Road**
**Brentford Middlesex (GB)**

(72) Inventor: **Clarke, Brian Peter**
**Coneybury Drive Spur Forest Drive**
**Kingswood, Surrey (GB)**
Inventor: **Stirling, Irene**
**38 Harrison Close**
**Reigate, Surrey (GB)**

(74) Representative: **Hesketh, Alan, Dr. et al,**
**European Patent Attorney Beecham Pharmaceuticals Great Burgh Yew Tree Bottom Road**
**Epsom, Surrey, KT18 5XQ (GB)**

**The file contains technical information submitted after the application was filed and not included in this specification**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 028 083 B1

# Derivatives of clavulanic acid, a process for their preparation and pharmaceutical compositions containing them

This invention relates to a novel class of clavulanic acid derivatives and in particular to a class of cyclic amine derivatives of clavulanic acid. These compounds have antibacterial and β-lactamase inhibitory qualities and, therefore, are of use in the treatment of bacterial infection either alone or in a synergistic composition with other antibacterial agents, such as penicillins or cephalosporins.

Our earlier British Patent Specification No. 1 566 706 (corresponding to French Patent No. 76—30454, DE—A1—2 646 003 and Japanese Patent Application No. 122727/76) disclosed inter alia the compounds of the formula (I):

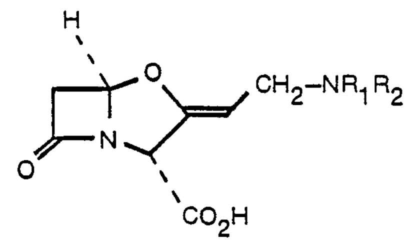

(I)

and salts and esters thereof, wherein $R_1$ is an inert organic group of up to 14 carbon atoms and $R_2$ is an inert organic group of up to 16 carbon atoms, the $NR_1R_2$ group containing up to 22 carbon atoms.

The present invention provides the compounds of the formula (II):

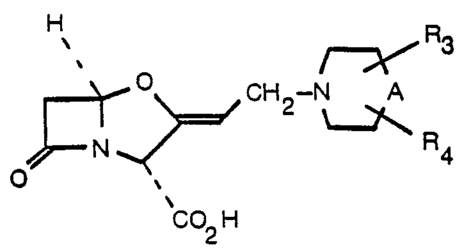

(II)

and pharmaceutically acceptable salts and esters thereof, wherein A is an oxygen or sulphur atom or is a group $NR_5$, wherein $R_5$ is a hydrogen atom or a $C_{1-6}$ alkyl, $C_{1-6}$ alkanoyl, $C_{1-6}$ alkoxycarbonyl, arylcarbonyl or aralkylcarbonyl group, any of such groups being optionally substituted by $C_{1-3}$ alkyl, $C_{1-3}$ alkoxy, hydroxy, $C_{1-3}$ alkanoyloxy, or esterified or salified carboxy; or $R_5$ is a group of the sub-formula (i):

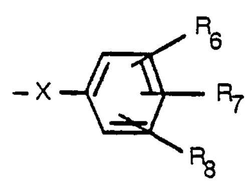

(i)

wherein X is a bond or a $—CH_2—$ moiety; $R_6$ is hydrogen, fluorine, chlorine, bromine, $C_{1-3}$ alkyl, $C_{1-3}$ alkoxy or hydroxy; $R_7$ is hydrogen or $C_{1-3}$ alkyl, and $R_8$ is hydrogen or $C_{1-3}$ alkyl; and $R_3$ and $R_4$ are independently hydrogen or $C_{1-6}$ alkyl.

The compounds of this invention have improved β-lactamase inhibitory properties compared to the compounds of British Patent 1,566,706.

Suitably A is an oxygen or sulphur atom, of which the oxygen atom is preferred. More suitably A is a $NR_5$ group.

Suitably $R_3$ is a hydrogen atom. Suitably also $R_3$ is a $C_{1-4}$ alkyl group, for example a methyl group. Suitably $R_4$ is a hydrogen atom. Suitably also $R_4$ is a $C_{1-4}$ alkyl group, for example a methyl group. In a preferred aspect $R_3$ and $R_4$ are independently hydrogen atoms.

Aptly $R_5$ is a hydrogen atom or an optionally substituted $C_{1-6}$ alkyl group, for example $R_5$ may be hydrogen, $C_{1-6}$ alkyl, hydroxy $C_{1-6}$ alkyl or $C_{1-3}$ alkoxy $C_{1-6}$ alkyl. In a preferred aspect $R_5$ is hydrogen, methyl, ethyl, propyl.

In an alternative embodiment $R_5$ may be optionally substituted $C_{1-6}$ alkanoyl, $C_{1-6}$ alkoxycarbonyl, arylcarbonyl or aralkylcarbonyl. Examples of suitable aryl groups for inclusion in the group $R_5$ are naphthyl, pyrrolyl, furyl, thienyl, indolyl, thionaphthyl, benzofuryl and phenyl. Preferred values of $R_5$ include benzoyl, formyl, acetyl, propionyl, methoxycarbonyl, ethoxycarbonyl, propoxycarbonyl, phenylacetyl and hydroxyphenylacetyl, of these formyl, benzoyl and acetyl are particularly favoured.

Suitably also $R_5$ is a group of the sub-formula (ii):

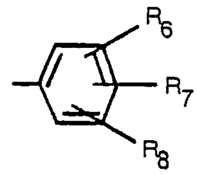

(ii)

wherein $R_6$, $R_7$ and $R_8$ are as hereinabove defined. More suitably $R_6$ is hydrogen, fluorine, chlorine, bromine, methyl or methoxy. More suitably $R_7$ and $R_8$ are independently hydrogen atoms. In a preferred aspect $R_6$, $R_7$ and $R_8$ are all independently hydrogen atoms.

Suitably also $R_5$ is a group of the sub-formula (iii):

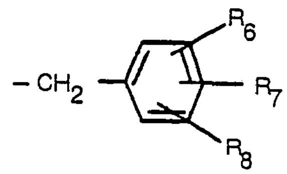

(iii)

wherein $R_6$, $R_7$ and $R_8$ are as hereinabove defined. More suitably $R_6$ is hydrogen, methyl or methoxy. More suitably $R_7$ and $R_8$ are independently hydrogen atoms. In a preferred aspect $R_6$, $R_7$ and $R_8$ are all independently hydrogen atoms.

The compounds of this invention are preferably in the form of the free acid of the formula (II), and as such exist in the form of a zwitterion, that is they may be represented as shown in formula (III):

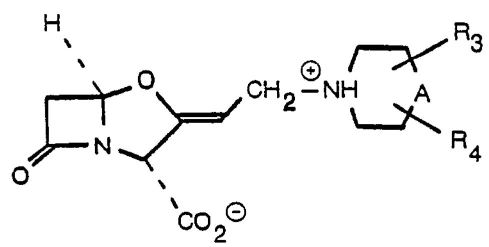

(III)

wherein A, $R_3$ and $R_4$ are as defined in relation to formula (II). These zwitterionic forms of the compounds are favoured in view of their crystalline form, stability and good solubility.

Other salts of the compounds of the formula (II) include those with pharmaceutically acceptable salting-ions, such as alkali metal and alkaline earth metal ions, for example the sodium, potassium and calcium ions; but such salts are not a preferred feature of this invention.

Thus preferred compounds of this invention include: benzyl 9-(4-benzylpiperazin-1-yl)deoxyclavulanate, 9 - (4 - benzylpiperazin-1-yl)deoxyclavulanic acid, benzyl 9 - (4 - ethoxycarbonylpiperazin-1-yl)deoxyclavulanate, 9 - (4 - ethoxycarbonylpiperazin - 1 - 1yl)deoxyclavulanic acid, benzyl 9 - (4-formylpiperazin - 1 - yl)deoxyclavulanate, 9 - (4 - formylpiperazin - 1 - yl)deoxyclavulanic acid, benzyl 9 - (thiomorpholino)deoxyclavulanate, p - nitrobenzyl 9 - (thiomorpholino)deoxyclavulanate, 9-4(thiomorpholino)deoxyclavulanic acid, benzyl 9 - (morpholino)deoxyclavulanate, 9 - (morpholino)-deoxyclavulanic acid, benzyl 9 - (2,6 - dimethylmorpholino)deoxyclavulanate, 9 - (2′,6′ - dimethylmorpholino)deoxyclavulanic acid, benzyl 9 - (4 - phenylpiperazin - 1 - yl)deoxyclavulanate, and 9 - (4-phenylpiperazin-1-yl)deoxyclavulanic acid.

Esters of the compounds of the formula (II) also form part of this invention, for example as the free base or as the acid addition salt since such compounds also can be used to enhance the effectiveness of penicillins and cephalosporins.

Suitable esters of the compounds of the formula (II) includes those of the formula (IV):

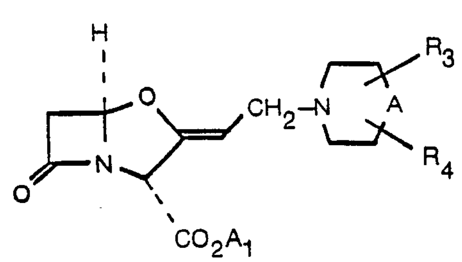

(IV)

wherein $A_1$ is an alkyl group of 1—6 carbon atoms optionally substituted by an alkoxy or acyloxy group of 1—7 carbon atoms, or is a group of the sub-formula (a):

$$CHA_2A_3 \quad (a)$$

wherein $A_2$ is an alkenyl or alkynyl group of up to 5 carbon atoms or is a phenyl group optionally substituted by a fluorine, chlorine, bromine, nitro or alkyl or alkoxyl of up to 4 carbon atoms; and $A_3$ is a hydrogen atom, an alkyl group of up to 4 atoms or a phenyl group optionally substituted by a fluorine, chlorine, bromine, nitro or alkyl or alkoxyl of up to 4 carbon atoms.

Suitable values for $A_1$ include methyl, ethyl, n-propyl, n-butyl, allyl, 2-methylallyl, propargyl, methoxymethyl, acetoxymethyl, propionoxymethyl, pivaloyloxymethyl, ethoxycarbonyloxymethyl, methoxycarbonyloxyethyl, ethoxycarbonyloxyethyl, phthalidyl, dimethoxyphthalidyl, benzyl, methoxybenzyl, ethoxybenzyl, nitrobenzyl and chlorobenzyl groups.

Certain favoured values $A_1$ include methyl, ethyl, propyl and methoxymethyl.

Certain favoured groups $A_2$ include the phenyl and 4-methoxyphenyl groups. A particularly favoured moiety $A_3$ is the hydrogen atom.

Certain other favoured values for $A_1$ include those of the sub-formulae (b), (c) and (d):

$$—CHA_4—OA_5 \quad (b)$$

$$—CHA_4—COA_5 \quad (c)$$

$$—CHA_4—OCOA_5 \quad (d)$$

wherein $A_4$ is a hydrogen atom or a methyl group and $A_5$ is an alkyl group of up to 4 carbon atoms or a phenyl or benzyl group either of which may be substituted by one or two alkyl or alkoxyl groups of up to 3 carbon atoms or by a fluorine, chlorine or bromine atom or a nitro group.

An apt acyclic value for the sub-group of the formula (b) is $—CH_2OA_5$.

An apt acyclic value for the sub-group of the formula (c) is $—CH_2—CO—A_5$.

An apt acyclic value for the sub-group of the formula (d) is $—CH_2—CO_2A_5$.

A further apt acyclic value for the sub-group of the formula (d) is $—CH(CH_3)—CO_2A_5$.

Favoured values for $A_5$ in the preceding acyclic moieties include the methyl, ethyl, propyl, butyl, phenyl and benzyl groups.

Esters of the compounds of the formula (II) such as those of the compounds of the formula (IV) may be presented in the form of their acid addition salts if desired. The acid used to form the salt will most suitably be pharmaceutically acceptable, but non-pharmaceutically acceptable acid addition salts are also envisaged, for example as intermediates in the preparation of the pharmaceutically acceptable salts by ion exchange. Suitable pharmaceutically acceptable acid additic salts include those of inorganic and organic acids, such as hydrochloric, phosphoric, sulp ric, methanesulphonic, toluenesulphonic, citric, malic, acetic, lactic, tartaric, propionic and succin. acid.

Most suitably the acid addition salt is provided as a solid and preferably as a crystalline lid.

Compounds of this invention when in crystalline form may be solvated, for example hydra.ed.

The present invention provides a pharmaceutical composition which comprises a compound of this invention and a pharmaceutically acceptable carrier.

The compositions of the invention include those in a form adapted for oral, topical or parenteral use and may be used for the treatment of the infection in mammals including humans.

Suitable forms of the compositions of this invention include tablets, capsules, creams, syrups, suspensions, solutions, reconstitutable powders and sterile forms suitable for injection or infusion. Such compositions may contain conventional pharmaceutically acceptable materials such as diluents, binders, colours, flavours, preservatives, disintegrant and the like in accordance with conventional pharmaceutical practice in the manner well understood by those skilled in the art of formulating antibiotics.

Injectable or infusable compositions of a compound of the invention are particularly suitable as high blood levels of the compound can occur after administration by injection or infusion. Thus, one preferred composition aspect of this invention comprises a compound of the invention in sterile form and most suitably in sterile crystalline form. The zwitterionic compounds of this invention are particularly suitable for use in such compositions.

The injectable solution of the compound of this invention may be made up in a sterile pyrogen-free liquid such as water, aqueous ethanol or the like.

An alternative approach to administering the compounds of this invention and especially those zwitterionic compounds of the formula (II) is to utilise an injectable suspension. Such suspensions may be made up in sterile water; sterile saline or the like and may also contain suspending agents such as polyvinylpyrrolidone, lecithin or the like (for example in the manner described for amoxycillin trihydrate in Belgian Patent No 839109). Alternatively such compositions may be prepared in an acceptable oil suspending agent such as arachis oil or its equivalent. The use of suspensions can give rise to advantageously prolonged blood levels of the medicament. Belgian Patent No 839109 may be consulted for suitable methods and materials for producing injectable aqueous suspensions. For use in

such suspensions the zwitterionic compound of this invention should be in the form of fine particles as described in said Belgian Patent.

Unit dose compositions comprising a compound of this invention adapted for oral administration form a further suitable composition aspect of this invention.

Unit dose compositions comprising a compound of this invention adapted for topical administration are also presented by this invention. In this instance 'topical administration' also includes local administration to internal surfaces of mammary glands of cattle, for example during the treatment of mastitis by intra-mammary administration.

The compound of the formula may be present in the composition as sole therapeutic agent or it may be present together with other therapeutic agents such as a penicillin or cephalosporin. Considerable advantages accrue from the inclusion of a penicillin or cephalosporin since the resulting composition shows enhanced effectiveness (synergy).

Suitable penicillins for inclusion in the compositions of this invention include benzylpenicillin, phenoxymethylpenicillin, carbenicillin, azidocillin, propicillin, ampicillin, amoxycillin, epicillin, ticarcillin, cyclacillin, pirbenicillin, azlocillin, mezlocillin, celbenicillin, and other known penicillins including prodrugs therefore such as their *in vivo* hydrolysable esters such as the acetoxymethyl, pivaloyloxymethyl, $\alpha$-ethoxycarbonyloxyethyl or phthalidyl esters of ampicillin, benzylpenicillin or amoxycillin, and aldehyde or ketone adducts of penicillins containing a 6-$\alpha$-aminoacetamide side chain (such as hetacillin, metampicillin and analogous derivatives of amoxycillin) or $\alpha$-esters of carbenicillin or ticarcillin such as their phenyl or indanyl $\alpha$-esters.

Suitable cephalosporins for inclusion in the compositions of this invention include cefatrizine, cephaloridine, cephalothin, cefazolin, cephalexin, cephacetrile, cephamandole nafate, cephapirin, cephradine, 4-hydroxycephalexin, cefaparole, cephaloglycin, and other known cephalosporins or prodrugs thereof.

Such compounds are frequently used in the form of a salt or hydrate of the like.

Naturally if the penicillin or cephalosporin present in the composition is not suitable for oral administration then the composition will be adapted for parenteral administration.

Highly favoured penicillins for use in the compositions of this invention include ampicillin, amoxycillin, carbenicillin and ticarcillin. Such penicillins may be used as a pharmaceutically acceptable salt such as the sodium salt. Alternatively the ampicillin or amoxycillin may be used in the form of fine particles of the zwitterionic form (generally as ampicillin trihydrate or amoxycillin trihydrate) for use in an injectable suspension, for example, in the manner hereinbefore described for a compound of this invention.

The preferred penicillin for use in the synergistic composition is amoxycillin, for example as its sodium salt or trihydrate.

Particularly suitable cephalosporins for use in the compositions of this invention include cephaloridine and cefazolin which may be in the form of a pharmaceutically acceptable salt for example the sodium salt.

When present together with a cephalosporin or penicillin, the ratio of a compound of the invention to the penicillin or cephalosporin agent may vary over a wide range of ratios, such as from 10:1 to 1:10 for example about 3:1, 2:1, 1:1, 1:2, 1:3, 1:4, 1:5 or 1:6, (wt/wt, based on pure free antibiotic equivalent). Orally administrable compositions containing a compound of the invention will normally contain relatively more synergist than corresponding injectable compositions.

The total quantity of a compound of the invention in any unit dosage form will normally be between 25 and 1000 mg and will usually be between 50 and 500 mg, for example about 62.5, 100, 125, 150, 200 or 250 mg.

Compositions of this invention may be used for the treatment of infections of *inter alia*, the respiratory tract, the urinary tract and soft tissues in humans and mastitis in cattle.

Normally between 50 and 1000 mg of the compounds of the invention will be administered each day of treatment but more usually between 100 and 750 mg of the compounds of the invention will be administered per day, for example at 1—6 doses, more usually as 2, 3 or 4 doses.

The penicillin or cephalosporin in the synergistic composition of this invention will normally be present at approximately the amount at which it is conveniently used which will usually be expected to be from about 62.5 to 1000 mg per dose, more usually about 125, 250 or 500 mg per dose.

One particularly favoured composition of this invention will contain from 150 to 1000 mg of amoxycillin as the trihydrate or sodium salt and from 25 to 500 mg of a compound of this invention.

Most suitably this form of composition will contain a compound of the formula (II) when in crystalline zwitterionic form.

A further particularly favoured composition of this invention will contain from 150 to 1000 mg of ampicillin or a pro-drug therefore and from 25 to 500 mg of a compound of this invention.

Most suitably this form of composition will contain ampicillin trihydrate, ampicillin anhydrate, sodium ampicillin, hetacillin, pivampicillinhydrochloride, bacampicillin hydrochloride, or talampicillin hydrochloride. Most suitably this form of the composition will contain a compound of the formula (II) when in crystalline zwitterionic form.

Most suitably the preceding composition will contain from 200 to 700 mg of the penicillin

component. Most suitably the preceding composition will comprise from 50 to 250 mg of a compound of the formula (II) preferably in crystalline zwitterionic form.

Such compositions may be adapted for oral or parenteral use except when containing an *in vivo* hydrolysable ester of ampicillin or amoxycillin in which case the compositions will not be adapted for parenteral administration.

Another particularly favoured composition of this invention will contain from 200 to 2000 mg of carbenicillin, ticarcillin or a pro-drug therefore and from 50 to 500 mg of a compound of the invention.

Suitably this form of composition will contain di-sodium carbenicillin. Suitably this form of the composition will contain di-sodium ticarcillin.

More suitably this form of the composition will contain from 75 to 250 mg of a compound of the formula (II) preferably in crystalline zwitterionic form. Such compositions containing di-salts of carbenicillin and ticarcillin will be adapted for parenteral administration.

The present invention also provides a method of treating bacterial infections in humans or domestic mammals which comprises the administration of a composition of this invention.

Commonly the infection treated will be due to a strain of *Staphylococcus aureus, Klebsiella aerogenes, Escherichia coli,* or *Proteus sp.* The organisms believed to be most readily treated by an antibacterially effective amount of a compound of this invention is *Staphylococcus aureus.* The other organisms named are more readily treated by using a synergistically effective amount of the compound of the invention and a penicillin or cephalosporin. The administration of the two components may take place separately but in general we prefer to use a composition containing both the synergist and the penicillin or cephalosporin.

The indications for treatment include respiratory tract and urinary tract infections in humans and mastitis in cattle.

The present invention also provides a process for the preparation of a compound of the formula (II) or a pharmaceutically acceptable salt or ester thereof which process comprises the reaction of a compound of the formula (V):

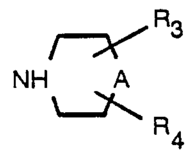

(V)

wherein A, $R_3$ and $R_4$ are as hereinbefore defined, with either an ester of a compound of the formula (VI) or a compound of the formula (VII) or salt or ester thereof:

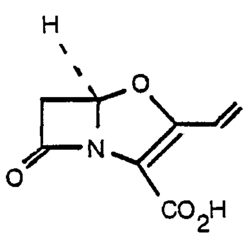

(VI)

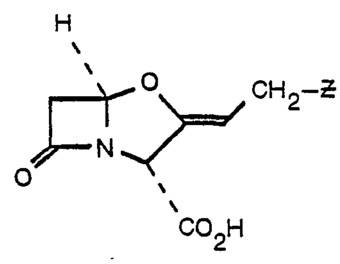

(VII)

wherein Ƶ is a displaceable group, and thereafter, if necessary, carrying out one or more of the following:

i) converting an ester, if present, to a free acid or a salt or another ester,

ii) converting the free acid or salt to an ester,

iii) forming an acid addition salt of any such ester.

Suitably Ƶ is a halide moiety, such as bromine, chloride or iodide. Suitably also Ƶ is a sulphonyloxy moiety, for example alkyl or aryl-sulphonyloxy, such as a mesylate or tosylate, or Ƶ is a carboxylate moiety, for example a $C_{1-6}$ alkanoyloxy group optionally substituted by 1 to 3 halogen atoms, in particular acetoxy, dichloroacetoxy and trichloroacetoxy.

Suitably the reaction of the compound of the formula (V) with the compounds of the formula (VI) or (VII) will occur in an aprotic solvent, such as dimethylformamide or acetonitrile, at a non-extreme temperature for example between −50°C and +50°C, more usually −40°C and +20°C, and most conveniently between −30°C and 0°C.

De-esterification of an ester of the compound of the formula (II) may be performed using conventional mild methods, such as hydrogenolysis and hydrolysis. In one aspect de-esterification is performed by hydrogenation using a low or medium pressure of hydrogen, for example one atmosphere, at an approximately ambient temperature in a conventional inert solvent. Favoured hydrogenolysable esters include allyl, substituted allyl, benzyl and substituted benzyl esters, for example the benzyl, 2-methylallyl, p-methoxybenzyl and p-nitrobenzyl esters. If a base is present in the

hydrogenation medium then a base salt may be formed. Normally no base is present and the zwitterionic salt results.

An alternative method of hydrogenation suitable for use in this procedure is catalytic transfer hydrogenation using a transition metal catalyst. The transition metal catalyst employed is preferably palladium, for example palladium on carbon, palladium on barium sulphate or palladium black. It is preferred to use palladium on carbon, for example 10% palladium on charcoal. Suitable solvents include those in which the ester is soluble, for example ethanol, dimethylformamide, dimethylacetamide and mixtures thereof. In catalytic transfer hydrogenation a hydrogen donor is used (not hydrogen gas). Suitable hydrogen donors include cyclohexene and 1,4-cyclohexadiene. The reaction is conveniently performed at an elevated temperature suitably between ambient and 100°C, more suitably between 30°C and 80°C. It is sometimes suitable to perform the reaction at reflux temperature.

When de-esterification is brought about by hydrolysis such as mild basic hydrolysis suitable base hydrolysable esters include the methoxymethyl and methyl esters, which undergo hydrolysis when maintained in an aqueous medium at a pH of 7 to 8.5. Such reactions can occur rapidly, for example 10—60 minutes. Most suitably such reactions take place in a solvent which is water or water together with an organic solvent, such as tetrahydrofuran. The reaction usually occurs sufficiently rapidly at 5°—20°C. The pH may be maintained at the correct level by the careful addition of base. This is generally a less suitable method of cleaving the ester than hydrogenolysis.

The present invention also provides a process for the preparation of an ester of a compound of the formula (II) which process comprises the reaction of the compound of the formula (II) with an esterifying agent.

The zwitterionic compound of the formula (II) may be dissolved or suspended in a solvent, such as dimethylformamide, hexamethylphosphoramide, dichloromethane, ethyl acetate or other non-esterifiable solvents and therein esterified. Suitable temperatures for such a reaction range from about 0° to about 25°C. Suitable esterifying reagents include reactive halides and their equivalents, alkyl oxonium salts and the like.

When a reagent such as a reactive iodide, chloride, bromide, tosylate, mesylate or the equivalent is used, the resulting salt is generally suitable for use in a composition of this invention. Alternatively, the salt may be converted to a free base or alternative salt. When an alkyl oxonium salt is used, it is preferred to convert the resulting tetrafluoroborate to the free base or alternative salt. The various aforementioned salts may be converted to the free base by neutralisation, for example by contacting a solution of the salt in water with an organic phase, neutralising the salt by adding a base and extracting the liberated amine into the organic phase. This amine may thereafter be re-salted by reacting with an appropriate acid, for example in a dry organic solvent. It is generally preferred to use not more than one equivalent of acid for this process. Alternatively, the originally formed salt may be converted into the alternative salt using an ion exchange material, for example by passing an aqueous solution of one salt through a bed of an anion exchange resin in the form of the desired salt such as the chloride form.

The salts may normally be obtained in solid form by dissolving in a fairly polar organic solvent (such as ethanol, tetrahydrofuran or the like) and then precipitating using a non-polar solvent, such as diethyl ether, cyclohexane or the like.

The salts of the esters of the compounds of the formula (II) may normally be obtained in crystalline form by conventional methods, such as trituration under (or crystallisation or recrystallisation from) a suitable organic solvent, such as ether, acetone, acetonitrile, tetrahydrofuran or the like.

The acid addition salt may be formed in situ or may be preformed. The acid employed will normally be a strong acid such as a methane sulphonic acid, p-toluene sulphonic acid or the like or trifluoroacetic acid or the like.

The reaction is normally carried out in an inert organic solvent. When the ester being formed is that of a liquid alcohol it is convenient to use that alcohol as the solvent or as part of the solvent system. The esterification is generally performed at a non-extreme temperature, such as 0°—35°C, for example from about 10°—25°C. Conveniently the reaction mixture may be performed at ambient temperature.

The following Examples serve to illustrate the invention.

Example 1

Benzyl 9-(4-benzylpiperazin-1-yl)deoxyclavulanate

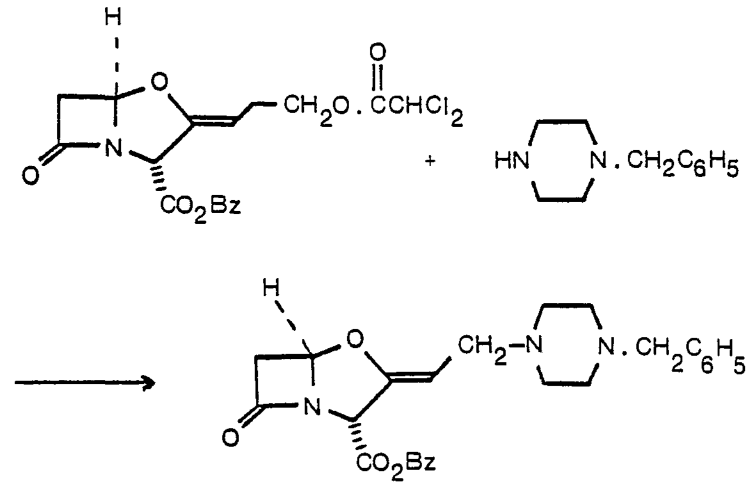

A solution of 1-benzylpiperazine (6.68 g; 38 mmol) in dimethylformamide was added dropwise to benzyl dichloroacetylclavulanate (8 g; 20 mmol) in dimethylformamide, at 0°. The reaction mixture was stirred at this temperature for 3 hours; poured into ethyl acetate (250 ml), washed with water (2 x 50 ml), brine (2 x 50 ml), dried (magnesium sulphate) and evaporated. The residual yellow oil was chromatographed on silica gel and the product eluted with methyl acetate. Fractions containing the product Rf ($SiO_2$/methyl acetate) = 0.3 were combined and evaporated to a colourless oil, yield 9.8%. $\nu$ ($CDCl_3$) 1800, 1745, 1703 $cm^{-1}$; $\delta$ ($CDCl_3$) 2.43 (8H, s, piperazine ring protons), 2.98 (1H, d, *J* 17 Hz, 6$\beta$-C*H*; partially obscured by d at $\delta$ 3.06), 3.06 (2H, d, *J* 7 Hz, 9-$CH_2$), 3.45 (1H, dd, *J* 17 and 3 Hz, 6$\alpha$-C*H*), 3.5 (2H, s, N . $CH_2C_6H_5$), 4.7 (1H, dt, *J* 7 and 1.6 Hz, 8-C*H*), 5.03 (1H, d, *J* 1.5 Hz, 3-C*H*), 5.16 (2H, s, $CO_2CH_2C_6H_5$), 5.63 (1H, dd, *J* 3 and 1.5 Hz, 5-C*H*), 7.27, 7.30 (10H, 2 x s, Ar-*H*).

Example 2

9-(4-Benzylpiperazin-1-yl)deoxyclavulanic acid

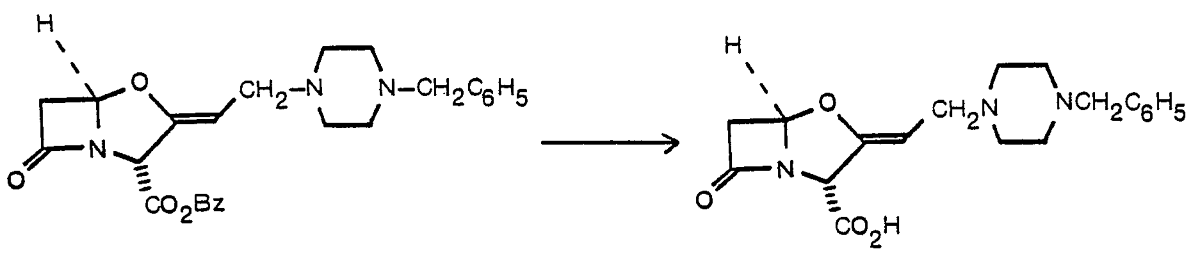

A solution of benzyl 9-(4-benzylpiperazin-1-yl)deoxyclavulanate (800 mg) in ethanol (50 ml) was hydrogenated at N.T.P. using 10% palladium on charcoal (200 mg) as catalyst. The catalyst was filtered off on Celite and the filtrate evaporated. The residue was chromatographed on cellulose using n-butanol: propan-2-ol: water, 4:4:1, as the eluting solvent. Fractions containing the product were combined and evaporated and the residue triturated with acetone/ether to give the title compound as a white solid in 20% yield. Rf ($SiO_2$/n-butanol: propan-2-ol: $H_2O$, 7:7:6) = 0.38; $\nu_{max}$ (KBr) 3400, 2940, 2810, 1785, 1688, 1615 $cm^{-1}$; $\delta$ ($D_2O$) 2.98 (9H, m, piperazine ring protons, 6$\beta$-C*H*), 3.55 (1H, dd, *J* 17 and 3 Hz, 6$\alpha$-C*H*; partially obscured by d at $\delta$ 3.61), 3.61 (2H, d, *J* 8 Hz, 9-$CH_2$), 3.76 (2H, s, N—$CH_2C_6H_5$), 4.73 (1H, bt, *J* 8 Hz, 8-C*H*), 4.95 (1H, bs, 3-C*H*), 5.69 (1H, d, *J* 3 Hz, 5-C*H*), 7.36 (5H, s, Ar-*H*).

Demonstration of Effectiveness

By a standard microtitre technique the MIC values for ampicillin alone, ampicillin and 9-(4-benzyl piperazinyldeoxyclavulanic acid and 9-(4-benzyl)piperazin-1-yldeoxyclavulanic acid alone were determined.

MIC $\mu g/ml$ (dilution in trytone soy broth)

| | Staph. aureus Russell | Kleb. aerogenes E70 | E. coli JT39 |
|---|---|---|---|
| Ampicillin alone | 1000 | 1000 | >2000 |
| Ampicillin + inhibitor | | | |
| at 1$\mu g/ml$ | 0.3 | 25 | 62 |
| 5$\mu g/ml$ | <0.01 | 3.1 | 16 |
| Inhibitor alone | 16 | 500 | >500 |

Antibacterial activity of 9-(4-benzylpiperazin-1-yl) deoxyclavulanic acid

| | |
|---|---|
| Staph. aureus Russell | 10 |
| Strep. pyogenes | 1.25 |
| Strep. pneumoniae | 5 |

Example 3

Benzyl 9-(4-ethoxycarbonylpiperazin-1-yl)deoxyclavulanate

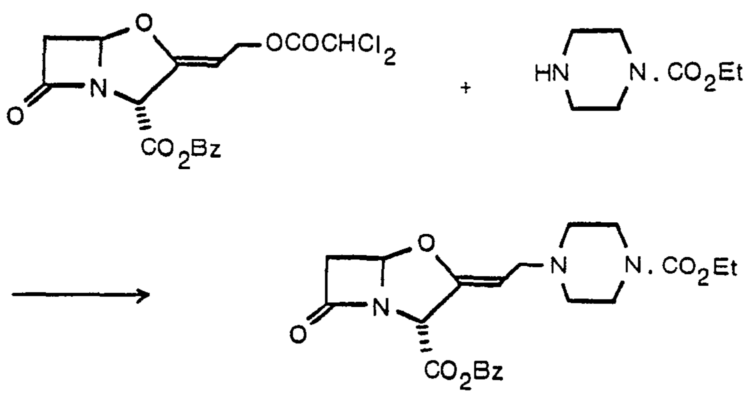

A solution of ethyl N-piperazinocarboxylate (4.26 g; 0.027 mol) in dimethylformamide (20 ml) was added slowly dropwise to a stirred solution of benzyl dichloroacetylclavulanate (6 g; 0.015 mol) at −30°C. When addition was complete stirring was continued at −20°C for 1.25 hr. The reaction mixture was poured into ethyl acetate (300 ml), washed with water (2 x 100 ml), dried ($MgSO_4$) and evaporated to an oil. The crude material was chromatographed on silica-gel eluting with methyl acetate/cyclohexane, 2:1. Fractions containing the product (Rf = 0.35, $SiO_2$/methyl acetate) were combined and evaporated to a colourless oil (3%); $\nu_{max}$ (film) 1800, 1735, 1700, 1650 $cm^{-1}$ $M^+$ 429.1865; $C_{22}H_{27}N_3O_6$ requires 429.1899.

Example 4

9-(4-Ethoxycarbonylpiperazin-1-yl)deoxyclavulanic acid

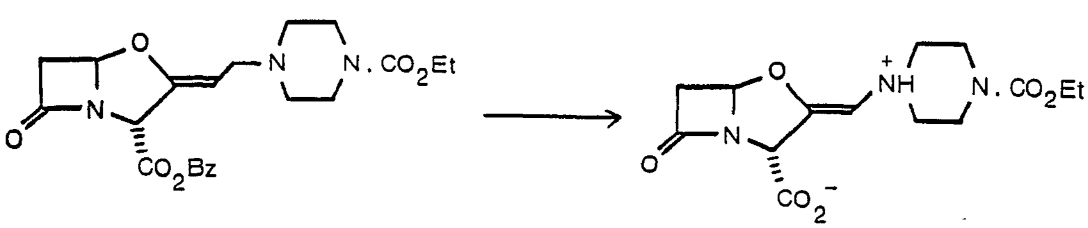

Benzyl 9-(4-ethoxycarbonylpiperazin-1-yl)deoxyclavulanate (160 mg) in tetrahydrofuran/water

(20 ml; 10:1) was hydrogenolysed at ambient pressure using 10% palladium on carbon (50 mg) as catalyst. After 45 minutes the catalyst was filtered off and the filtrate was evaporated to an oil. Addition of cold acetone precipitated the product as a white solid in 40% yield; $\nu_{max}$ (nujol) 1790, 1695, 1620 $cm^{-1}$; $\nu_{max}$ (KBr) 1790, 1697, 1620 $cm^{-1}$; $\delta$ ($D_2O$) 1.21 (3H, t, J 7 Hz), $CH_2CH_3$), 2.78 (4H, bt, $N(CH_2CH_2)N.CO_2Et$), 3.06 (1H, d, J 17 Hz, 6β-C*H*), 3.5 (7H, m, 6α-C*H*, 9-$CH_2$, $N(CH_2CH_2)_2N.CO_2Et$), 4.12 (2H, q, $CH_2CH_3$), 4.79 (1H, bt, obscured by HOD peak, 8-C*H*), 4.98 (1H, d, 3-C*H*), 5.74 (1H, d, J 3 Hz, 5-C*H*).

## Example 5

Benzyl 9-(4-formylpiperazin-1-yl)deoxyclavulanate

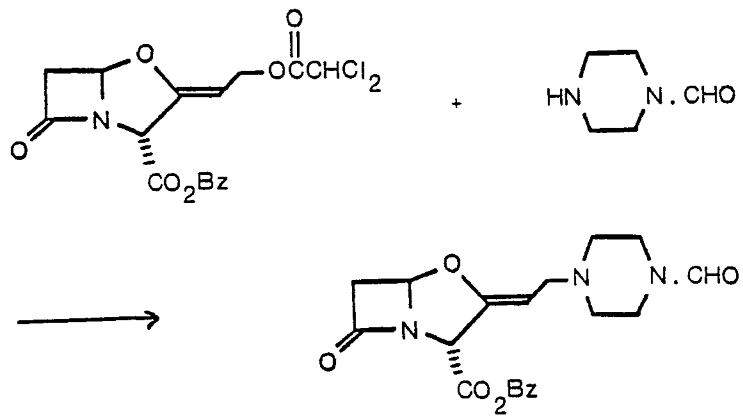

Benzyl dichloroacetylclavulanate (6 g; 0.015 mol) in dry dimethylformamide (60 ml) at −40° was treated, dropwise over 10 mins, with a solution of 1-piperazinecarboxaldehyde (3.1 g; 0.027 mol) in dimethylformamide (20 ml). The mixture was stirred at −20° for 2 hours, then poured into ethyl acetate (250 ml). The solution was washed with water then extracted with tartaric acid solution (20%), the acid extract was brought to pH 8.5 and back extracted with ethyl acetate. The organic phase was separated, washed with water, dried ($MgSO_4$) and evaporated to give an oil. $\nu_{max}$ (film) 1800, 1780, 1665 $cm^{-1}$.

## Example 6

9-(4-Formylpiperazin-1-yl)deoxyclavulanic acid

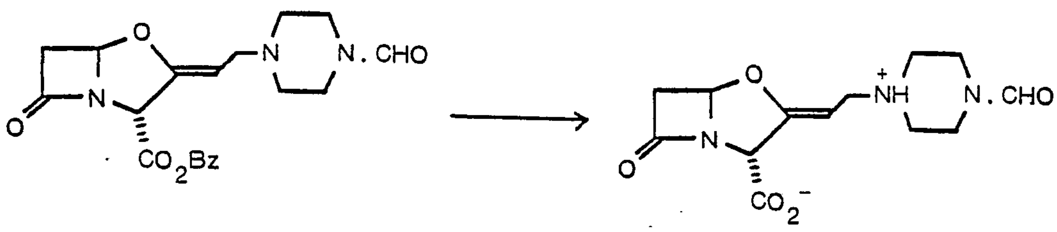

The crude ester from Example 5 was hydrogenolysed at ambient pressure in tetrahydrofuran:water, 10:1, for 45 mins, over 10% palladium on carbon. The catalyst was filtered off and the filtrate evaporated. Column chromatography on silica-gel, eluting with ethyl acetate:propan-1-ol:water, 5:4:4, gave the title compound as a white solid (acetonitrile) in <1% yield from benzyl dichloroacetylclavulanate Rf ($SiO_2$/ethyl acetate — propan-2-ol — water) = 0.16, $\nu_{max}$ (KBr) 1785, 1600—1700 (b) $cm^{-1}$; $\delta$ ($D_2O$) 2.62 (m, $N(CH_2CH_2)_2N.CHO$), 3.01 (d, 17 Hz, 6β-CH), 3.22 (d, J 8 Hz, 9-$CH_2$), 3.46 (m, 6α-C*H*, $N(CH_2CH_2)_2NCHO$), 4.75 (bt, partly obscured by HOD peak), 4.95 (bs, 3-C*H*), 5.68 (d, J 3 Hz, 5-C*H*), 7.95 (s, C*H*O).

## Example 7

Benzyl 9-(thiomorpholino)deoxyclavulanate

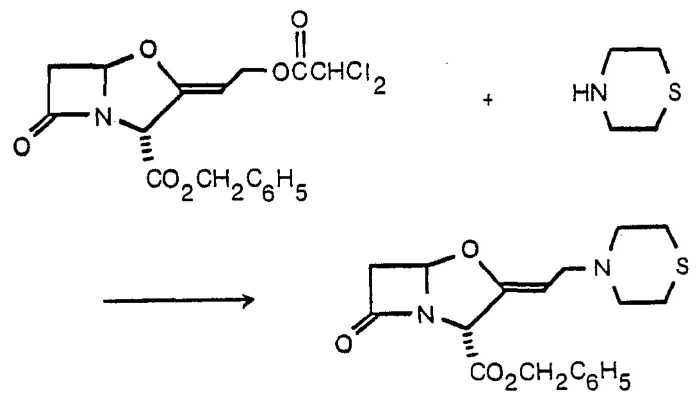

Benzyl dichloroacetylclavulanate (9.0 g; 22.5 m mol) in dry dimethylformamide (50 cm[3]) at —20° was treated with 1.9 equivalents of thiomorpholine (4.4 g) dropwise in dimethylformamide (20 cm[3]) and stirred for 35 minutes between —20° and —5°C. The mixture was poured into methylacetate (300 cm[3]) and washed with water (5 x 100 cm[3]), dried and evaporated to an oil. This oil was chromatographed on silica eluting with methylacetate:cyclohexane, 1:1. Fractions were collected containing the title compound Rf ($SiO_2$/methylacetate-cyclohexane, 1:1 = 0.5 (detection by aqueous potassium permanganate spray). Combined fractions were evaporated to afford 0.64 g of the title compound as an oil. ν (film) 1802, 1750, 1095, 742, 700 $cm^{-1}$, δ ($CDCl_3$), 2.61 (8H, bs, $N(CH_2CH_2)_2S$), 2.99 (1H, d, *J* 16, 6β*CH*), 3.05 (2H, d, *J* 7 Hz, $9CH_2$), 3.45 (1H, dd, *J* 16 and 3 Hz, 6α*CH*), 4.68 (1H, bt, J 7 Hz, 8*CH*), 5.06 (1H, bs, 3*CH*), 5.17 (2H, s, $OCH_2C_6H_5$), 5.65 (1H, d, *J* 3Hz, 5α*CH*), 7.42 (5H, s, $C_6H_5$).

## Example 8

p-Nitrobenzyl 9-(thiomorpholino)deoxyclavulanate

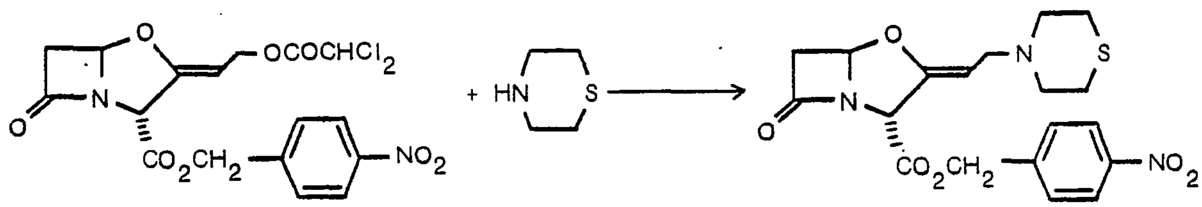

p-Nitrobenzyl dichloroacetylclavulanate (12.5 g; 28 mmol) in dry dimethylformamide (50 cm[3]) at —10° was treated with 1.9 equivalents of thiomorpholine in dimethylformamide (20 cm[3]), dropwise with stirring over several minutes. Stirring was continued for 1½ hours during which the temperature was allowed to rise to +10°. The solution was poured into ethyl acetate (200 cm[3]), and washed with water (3 x 100 cm[3]), saturated brine (5 x 100 cm[3]), dried (anhydrous magnesium sulphate) and evaporated to an oil. Toluene was added and filtered to remove some solid material. The filtrate was chromatographed on silica eluting with ethyl acetate — cyclohexane 1:2 grading to neat ethyl acetate. Fractions were collected containing the title compound Rf ($SiO_2$/ethyl acetate) = 0.65. Combined fractions were evaporated to afford a foam, 0.54 g (4.6%). ν (film) 1805, 1755, 1702, 1520, 847, 740 $cm^{-1}$, δ ($CDCl_3$) 2.64 (8H, bs, $N(CH_2CH_2)_2S$), 3.03 (1H, d, *J* 17 Hz, 6β*CH*), 3.10 (2H, d, *J* 7 Hz, $9CH_2$), 3.50 (1H, dd, *J* 17 and 3 Hz, 6α*CH*), 4.71 (1H, bt, *J* 7 Hz, 8*CH*), 5.11 (1H, bs, 3*CH*), 5.28 (2H, s, $CO_2CH_2$), 5.67 (1H, d, *J* 3 Hz, 5α*CH*), 7.50 (2H, d, *J* 9 Hz

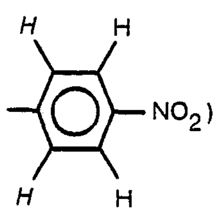), 8.24 (2H, d, *J* 9 Hz, 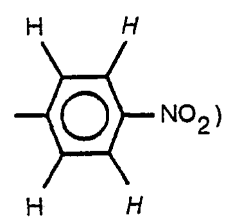).

## Example 9

9-4(Thiomorpholino)deoxyclavulanic acid

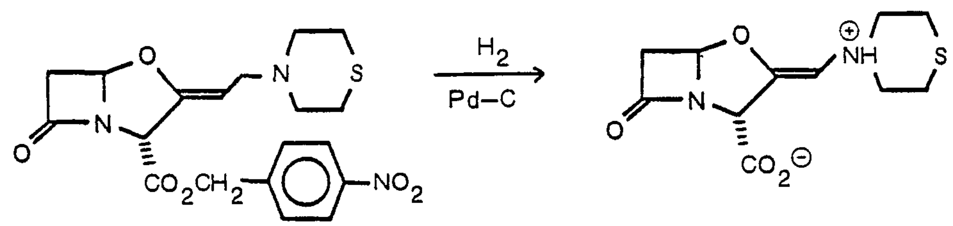


p-Nitrobenzyl 9-(thiomorpholino)deoxyclavulanate (0.45 g; 1.13 mmol) in tetrahydrofuran (15 cm$^3$), ethanol (5 cm$^3$) and water (2 cm$^3$) was hydrogenolysed at atmosphere pressure in the presence of 10% palladium on carbon (150 mg; which had been prehydrogenated for 10 minutes) for $1\frac{1}{2}$ hours. The catalyst was filtered off and the filtrate evaporated to an oil. This crude product was chromatographed on silica eluting with ethyl acetate — ethanol — water; 5:3:3. Fractions were collected containing the title compound Rf, ($SiO_2$/ethyl acetate — ethanol — water; 5:3:3) x 0.6, combined fractions were evaporated to afford 73 mg (23%) of a foam; $\nu$ (nujol) 1785, 1692, 1620 cm$^{-1}$; $\nu$ (KBr) 1785, 1692, 1615, 1380, 1308, 1190, 1117, 1042, 1017, 944, 898, 737 cm$^{-1}$. $\delta$ ($CD_3OD/D_2O$; 10:1) 2.70—2—95 (4H, broad m, $—\overset{H}{\underset{\oplus}{N}}(CH_2C\mathit{H}_2)_2S$), 3.00—3.25 (4H, broad m, $—\overset{H}{\underset{\oplus}{N}}(C\mathit{H}_2CH_2)_2S$), 6$\beta$CH obscured 3.47 (2H, d, $J$ 7 Hz, 9C$\mathit{H}_2$), 6$\alpha$CH obscured, 4.90 (1H, s, 3 C*H*), 8 CH obscured HOD, 5.75 (1H, d, $J$ 3 Hz 5$\alpha$C*H*).

## Example 10

Benzyl 9-(morpholino)deoxyclavulanate

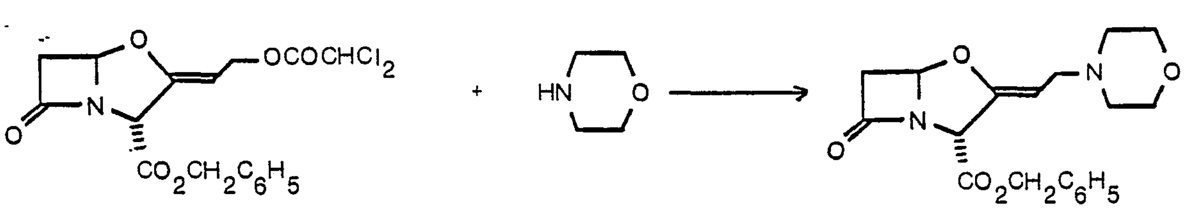

Benzyl dichloroacetylclavulanate (12.4 g; 31 mmol) in dry dimethylformamide (50 cm$^3$) at −30°C was treated with 1.9 equivalents of morpholine (5.1 cm$^3$) in dimethylformamide (30 cm$^3$) dropwise with stirring. Stirring was continued for 45 minutes during which the temperature was allowed to rise to 0°C. The mixture was poured into methyl acetate (300 cm$^3$) washed with water (5 x 100 cm$^3$), saturated brine (6 x 100 cm$^3$), dried with anhydrous magnesium sulphate and evaporated to an oil. This oil was chromatographed on silica eluting with methyl acetate — cyclohexane (2:1) grading to neat methyl acetate. Fractions were collected containing the title compound Rf ($SiO_2$/methyl acetate), = 0.35. Combined fractions were evaporated to afford an oil. This oil was dissolved in acetone and filtered cold to remove insoluble material. The filtrate was evaporated to afford 470 mg (4.2%) of an oil; $\nu$ (film) 1802, 1750, 1700, 745, 700 cm$^{-1}$ $\delta$ ($CDCl_3$) 2.2—2.45 (4H, broad m centred at $\delta$ 2.35, N(C$\mathit{H}_2$CH$_2$)$_2$O), 2.98 (1H, d, $J$ 17 Hz, 6$\beta$C*H*), 3.03 (2H, d, $J$ 7 Hz, 9 C$\mathit{H}_2$), 3.45 (1H, dd, $J$ 17 and 3 Hz, 6$\alpha$C*H*), 3.45—3.75 (4H, broad m centred at $\delta$ 3.63, N(CH$_2$C$\mathit{H}_2$)$_2$O), 4.68 (1H, broad t, $J$ 7 Hz, 8C*H*), 5.06 (1H, broad s, 3C*H*), 5.16 (2H, s, OC$\mathit{H}_2$C$_6$H$_5$), 5.65 (1H, d, $J$ 3Hz, 5$\alpha$C*H*), 7.33 (5H, s, CH$_2$C$_6\mathit{H}_5$).

## Example 11

9-(Morpholino)deoxyclavulanic acid

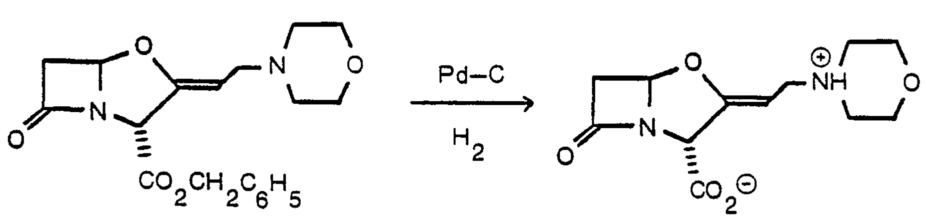


Benzyl 9-(morpholino)deoxyclavulanate (0.369; 1.03 mmol) in tetrahydrofuran (10 cm$^3$), water (2 cm$^3$) and 10% palladium on carbon was hydrogenolysed at atmospheric pressure for 30 minutes. The

catalyst was filtered off and evaporated to an oil. Ethanol (3 $cm^3$) was added, crystals slowly formed, when acetonitrile (2 $cm^3$) was added and the mixture cooled (−5°C). The crystals were filtered off, washed with ethanol and dried to afford 140 mg (51%) of the title compound. Rf ($SiO_2$/methylacetate-ethanol-water; 5:2:2) = 0.4 (detection by aqueous potassium permanganate spray, $\nu$ (nujol) (2700—1880), 1780, 1680, 1630, 1305, 1292, 1205, 1115, 1050, 1030, 1005, 985, 960, 910, 790, 742 $cm^{-1}$ $\delta$ (KBr) (2700—1900), 1785, 1682, 1630, 1455, 1410, 1370, 1350, 1305, 1292, 1205, 1117, 1047, 1040, 1004, 985, 960, 912, 790, 742 $cm^{-1}$, $\delta$ ($D_2O$) 3.08 (1H, d, *J* 17 Hz, 6$\beta$C*H*), 3.28 (4H, very broad m centred at 3.28, $N(CH_2CH_2)_2O$), 3.57 (1H, dd, *J* 17 and 3 Hz, 6$\alpha$C*H*), 3.80 (2H, d, *J* 8 Hz, $9CH_2$), 3.90 (4H, very broad m centred at 3.90, $N(CH_2CH_2)_2O$), 4.79 (1H, broad t, *J* 8 Hz, 8C*H*), 5.02 (1H, s, 3C*H*), 5.77 (1H, d, *J* 3 Hz, 5$\alpha$C*H*).

Example 12

Benzyl 9-(2,6-dimethylmorpholino)deoxyclavulanate

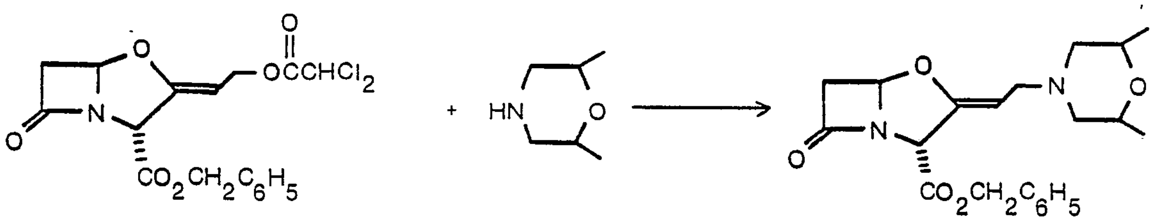

Benzyl dichloroacetylclavulanate (8 g; 20 mmol) in dry dimethylformamide (50 $cm^3$) at −30°C was treated with 1.9 equivalents of 2,6-dimethylmorpholine, dropwise in 20 $cm^3$ of dimethylformamide over 5 minutes with vigorous stirring. The mixture was stirred for 1 hour between −25° and −5°C. The mixture was poured into methylacetate (300 $cm^3$) and washed with water (5 × 100 $cm^3$), saturated brine (6 × 100 $cm^3$), dried (anhydrous magnesium sulphate) and evaporated in the presence of toluene to low volume, this crude product was chromatographed on silica eluting with methylacetate-cyclohexane (2:1) fractions were collected containing moderately pure material Rf ($SiO_2$/methylacetate-cyclohexane; 2.1? = 0.4 (detection by aqueous potassium permanganate spray). Combined fractions were evaporated and rechromatographed to afford the title compound as an oil, yield = 240 mg (3%) $\nu$ (film) 1805, 1750, 1700, 745, 700 $cm^{-1}$. The proton magnetic resonance spectrum was consistent with the desired compound.

Example 13

9-(2,6-Dimethylmorpholino)deoxyclavulanic acid

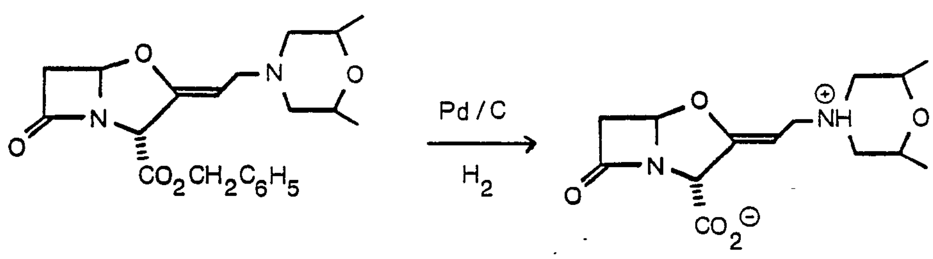


Benzyl 9-(2,6-dimethylmorpholino)deoxyclavulanate (0.168 g; 0.435 mmol) in tetrahydrofuran (10 $cm^3$) and water (2 $cm^3$) with 80 mg 10% palladium on carbon was hydrogenolysed at atmospheric pressure for 30 minutes. The catalyst was filtered off, washed with aqueous tetrahydrofuran and evaporated to an oil. This oil was chromatographed on silica eluting with methylacetate — ethanol — water; 5:2:2. Fractions were collected containing the title compound Rf ($SiO_2$/methyl — acetate — ethanol — water: 5:2:2) = 0.5 (detection by aqueous potassium permanganate spray). Combined fractions were evaporated to a foam from acetonitrile, yield = 63 mg (49%), $\nu$ (nujol) (3700—3120), (2550—2000), 1790, 1695, 1620, 1305, 1180, 1145, 1120, 1080, 1040, 1020, 998, 900, 740 $cm^{-1}$, $\nu$ (KBr) 1787, 1695, 1620, 1377, 1310, 1193, 1180, 1120, 1080, 1040, 1020, 996, 900, 740 $cm^{-1}$. $\delta$ ($D_2O$) internal standard $CH_3CN$ at $\delta$ 1.97, 1.11 (6H, d, *J* 6 Hz, 2 × $CHCH_3$), 2.27 (2H, dd, *J*-trans 11 and *J*-gem 12 Hz,

2 × 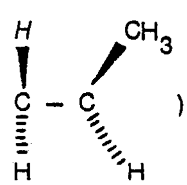 ),

3.01 (1H, d, *J* 17 Hz, 6$\beta$C*H*), 3.10 (2H, bd, *J*-gem 12 Hz,

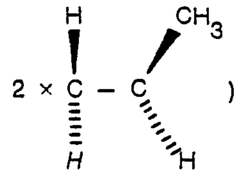

3.43 (2H, d, *J* 7 Hz, 9 $CH_2$), 3.49 (1H, dd, *J* 17 and 3 Hz, 6$\alpha$C*H*), 3.35—3.95 (2H, broad m, partially obscured.

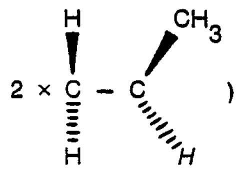

4.72 (1H, t, *J* 7Hz, 8C*H*), 4.92 (1H, bs, 3C*H*), 5.69 (1H, d, *J* 3 Hz, 5$\alpha$C*H*).

## Example 14

Benzyl 9-(4-phenylpiperazin-1-yl)deoxyclavulanate

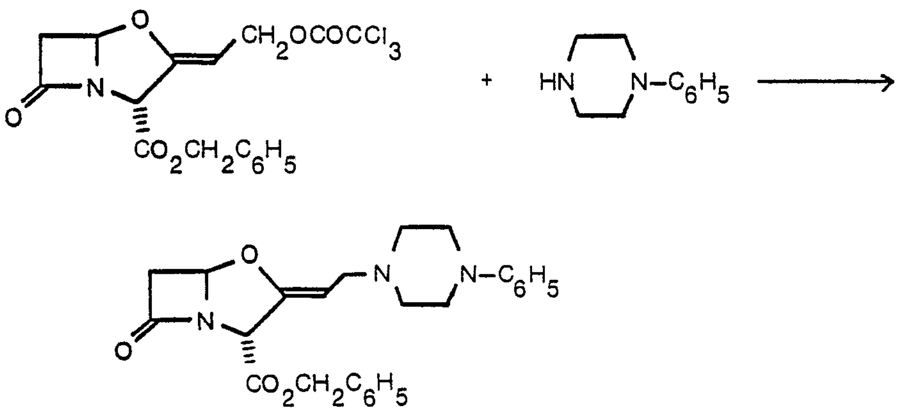

Benzyl trichloroacetylclavulanate (8.68 g; 20 mmol) in dry dimethylformamide (50 cm³) at −30°C was treated with 1.9 equivalents of N-phenylpiperazine (in 20 cm³ dimethylformamide) dropwise over 5 minutes, then stirred at −25° to −10° for 20 minutes. The mixture was poured into methylacetate (300 cm³) and washed with water (4 x 100 cm³), saturated brine (6 x 100 cm³), dried (anhydrous magnesium sulphate) and evaporated in the presence of toluene to low volume. This crude product was chromatographed on silica eluting with methylacetate/cyclohexane (3:1). Fractions were collected containing material Rf (methylacetate/cyclohexane, 2:1) = 0.5 (detection by aqueous potassium permanganate spray) combined fractions were evaporated to afford 900 mg of a foam. 400 mg of this moderately pure product was rechromatographed on silica eluting with methylacetate — cyclohexane 3:1. Fractions were collected containing the title compound and these were evaporated to afford 120 mg of a foam, (film) 1805, 1750, 1702, 1600, 1495, 1450, 1305, 1230, 1000, 750, 690 cm⁻¹. The proton magnetic resonance spectrum was consistent with the desired product.

## Example 15

9-(4-Phenylpiperazin-1-yl)deoxyclavulanic acid

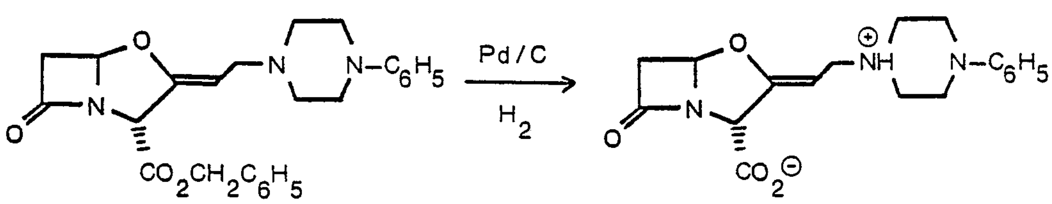


Benzyl 9-(4-phenylpiperazin-1-yl)deoxyclavulanate (0.37 g of moderately pure material) in tetrahydrofuran (10 cm³), ethanol (5 cm³) and water (2 cm³) was hydrogenolysed at atmospheric pressure in the presence of 150 mg of 10% palladium on carbon for 30 minutes. The catalyst was

filtered off and the filtrate evaporated to a foam. This crude product was chromatographed on silica eluting with methylacetate-ethanol-water (5:2:2), fractions were collected containing the title compound Rf ($SiO_2$/methylacetate-ethanol-water; 5:2:1) = 0.56 (detection by aqueous potassium permanganate spray), combined fractions were evaporated to a foam, yield = 63 mg (22%), $\nu$ (nujol) 1785, 1695, 1620, 1600, 1500, 1460, 1305, 1230, 1190, 1110, 1040, 1020, 995, 760, 740, 695 $cm^{-1}$, (KBr) 1790, 1695, (1675—1540 broad), 1497, 1455, 1380, 1308, 1230, 1192, 1110, 1040, 1020, 997, 760, 740, 692 $cm^{-1}$, $\delta$ ($CD_3OD/D_2O$) 3.01 (1H, d, $J$ 17 Hz 6$\alpha$C$H$, partially obscured), 3.0—3.75 (11H, m, N(C$H_2$C$H_2$)$_2$N; 9C$H_2$ at 3.6, $J$ 8Hz, 6$\alpha$C$H$), 8C$H$ partially obscured by HOD 4.93 (1H, s, 3C$H$), 5.78 (1H, d, $J$ 3 Hz, 5$\alpha$C$H$), 6.80—7.44 (5H, m, N$C_6H_5$).

Demonstration of Effectiveness

By a standard microtitre technique the following MIC values were obtained in μg/ml.

| Concentration of Inhibitor (μg/ml) | Amoxycillin alone | Amoxycillin + compound of Example 4 | | 8 | | 11 | | 13 | | 15 | |
|---|---|---|---|---|---|---|---|---|---|---|---|
| | | 5.0 | 1.0 | 5.0 | 1.0 | 5.0 | 1.0 | 5.0 | 1.0 | 5.0 | 1.0 |
| S. aureus Russell | 500 | 0.3 | 1.25 | – | 0.62 | 0.15 | 0.6 | 0.3 | 1.25 | – | 0.16 |
| K. aerogenes E70 | 500 | 1.5 | 6.25 | 1.6 | 3.1 | 1.5 | 3.1 | 1.5 | 6.2 | 6.2 | 12.5 |
| E coli JT39 | 500 | 4.0 | 31.2 | 2.0 | 8.0 | 4.0 | 16.0 | 4.0 | 31.2 | 4.0 | 31.2 |

The compounds of this invention alone had no activity at the above concentrations.

## Claims

1. A compound of the formula (II):

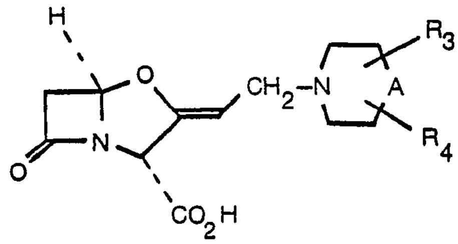

(II)

and pharmaceutically acceptable salts and esters thereof, wherein A is an oxygen or sulphur atom or is a group $NR_5$, wherein $R_5$ is a hydrogen atom or a $C_{1-6}$ alkyl, $C_{1-6}$ alkanoyl, $C_{1-6}$ alkoxycarbonyl, arylcarbonyl or aralkylcarbonyl group, any of such groups being optionally substituted by $C_{1-3}$ alkyl, $C_{1-3}$ alkoxy, hydroxy, $C_{1-3}$ alkanoyloxy, or esterified or salified carboxy; or $R_5$ is a group of the sub-formula (i):

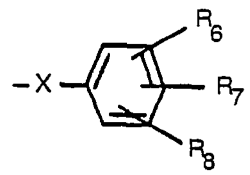

(i)

wherein X is a bond or a —$CH_2$— moiety; $R_6$ is hydrogen, fluorine, chlorine, bromine, $C_{1-3}$ alkyl, $C_{1-3}$ alkoxy or hydroxy; $R_7$ is hydrogen or $C_{1-3}$ alkyl, and $R_8$ is hydrogen or $C_{1-3}$ alkyl; and $R_3$ and $R_4$ are independently hydrogen or $C_{1-6}$ alkyl.

2. A compound as claimed in claim 1 wherein $R_3$ and $R_4$ are independently hydrogen atoms.

3. A compound as claimed in either claim 1 or claim 2, wherein $R_5$ is benzoyl, formyl, acetyl, propionyl, methoxycarbonyl, ethoxycarbonyl, propoxycarbonyl, phenylacetyl or hydroxyphenylacetyl.

4. A compound as claimed in either claim 1 or claim 2, wherein $R_5$ is phenyl, fluorophenyl, chlorophenyl, bromophenyl, methylphenyl or methoxyphenyl.

5. A compound as claimed in either claim 1 or claim 2, wherein $R_5$ is benzyl, fluorobenzyl, chlorobenzyl, bromobenzyl, methylbenzyl or methoxybenzyl.

6. A compound selected from: benzyl 9-(4-benzylpiperazin-1-yl)deoxyclavulanate, 9-(4-benzylpiperazin-1-yl)deoxyclavulanic acid, benzyl 9-(4-ethoxycarbonylpiperazin-1-yl)deoxyclavulanate, 9-(4-ethoxycarbonylpiperazin-1-yl)deoxyclavulanic acid, benzyl 9-(4 formylpiperazin-1-yl)deoxyclavulanate, 9-(4-formylpiperazin-1-yl)deoxyclavulanic acid, benzyl 9-(4-phenylpiperazin-1-yl)deoxyclavulanate, and 9-(4-phenylpiperazin-1-yl)deoxyclavulanic acid.

7. A compound selected from: benzyl 9-(thiomorpholino)deoxyclavulanate, p-nitrobenzyl 9-(thiomorpholino)deoxyclavulanate, 9-4-(thiomorpholino) deoxyclavulanic acid.

8. A compound selected from: benzyl 9-(morpholino)deoxyclavulanate, 9-(morpholino)deoxyclavulanic acid, benzyl 9-(2,6-dimethylmorpholino) deoxyclavulanate, 9-(2′,6′-dimethylmorpholino) deoxyclavulanic acid.

9. A compound as claimed in any of claims 1 to 8 when in zwitterionic form.

10. A pharmaceutical composition which comprises a compound as claimed in any of claims 1 to 9 and a pharmaceutically acceptable carrier.

11. A composition as claimed in claim 10 which also comprises a penicillin or cephalosporin.

12. A process for the preparation of a compound as claimed in claim 1 or a pharmaceutically acceptable salt or ester thereof, which process comprises the reaction of compound of the formula (V):

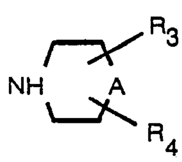

(V)

wherein A, $R_3$ and $R_4$ are as defined in claim 1, with either an ester of a compound of the formula (VI) or a compound of the formula (VII) or salt or ester thereof:

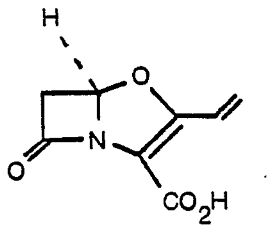

(VI)

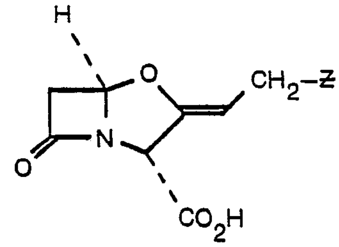

(VII)

wherein Z is a displaceable group, and thereafter, if necessary, carrying out one or more of the following:

i) converting an ester, if present, to a free acid or a salt or another ester,
ii) converting the free acid or salt to an ester,
iii) forming an acid addition salt of any such ester.

13. A process as claimed in claim 12, wherein Z is a dichloroacetoxy group.

14. A compound as claimed in any of claim 1 to 9 for use in the treatment of bacterial infection.

**Patentansprüche**

1. Eine Verbindung der Formel II

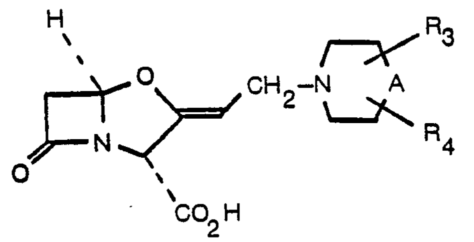

(II)

und pharmakologisch annehmbare Salze und Ester derselben, in welcher A ein Sauerstoff- oder Schwefelatom oder eine Gruppe $NR_5$ ist, in welcher $R_5$ ein Wasserstoffatom oder eine $C_{1-6}$-Alkyl-, $C_{1-6}$-Alkanoyl-, $C_{1-6}$-Alkoxycarbonyl-, Arylcarbonyl- oder Aralkylcarbonylgruppe ist, jede solcher Gruppen gegebenenfalls durch $C_{1-3}$-Alkyl, $C_{1-3}$-Alkoxy, Hydroxy, $C_{1-3}$-Alkanoyloxy oder eine veresterte oder in Salzform überführte Carboxylgruppe substituiert sein kann, oder $R_5$ eine Gruppe der Unterformel (i) ist:

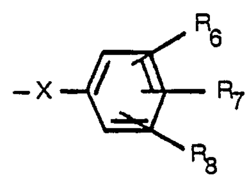

(i)

in welcher X eine Bindung oder ein Molekülteil —$CH_2$—, $R_6$ Wasserstoff, Fluor, Chlor, Brom, $C_{1-3}$-Alkyl, $C_{1-3}$-Alkoxy oder Hydroxy bedeutet, $R_7$ Wasserstoff oder $C_{1-3}$ Alkyl und $R_8$ Wasserstoff oder $C_{1-3}$-Alkyl bedeutet; und $R_3$ und $R_4$ unabhängig voneinander Wasserstoff oder $C_{1-6}$-Alkyl sind.

2. Eine Verbindung wie in Anspruch 1 beansprucht, in welcher $R_3$ und $R_4$ unabhängig voneinander Wasserstoffatome sind.

3. Eine Verbindung wie entweder in Anspruch 1 oder Anspruch 2 beansprucht, in welcher $R_5$ Benzoyl, Formyl, Acetyl, Propionyl, Methoxycarbonyl, Äthoxycarbonyl, Propoxycarbonyl, Phenylacetyl oder Hydroxyphenylacetyl ist.

4. Eine Verbindung wie entweder in Anspruch 1 oder Anspruch 2 beansprucht, in welcher $R_5$ Phenyl, Fluorphenyl, Chlorphenyl, Bromphenyl, Methylphenyl oder Methoxyphenyl ist.

5. Eine Verbindung wie entweder in Anspruch 1 oder Anspruch 2 beansprucht, in welcher $R_5$ Benzyl, Fluorbenzyl, Chlorbenzyl, Brombenzyl, Methylbenzyl oder Methoxybenzyl ist.

6. Eine Verbindung, ausgewählt aus: Benzyl-9-(4-benzylpiperazin-1-yl)deoxyclavulanat, 9-(4-Benzylpiperazin-1-yl)-deoxyclavulansäure, Benzyl-9-(4-äthoxycarbonylpiperazin-1-yl)deoxyclavulanat, 9-(4-Äthoxycarbonylpiperazin-1-yl)deoxyclavulansäure, Benzyl-9-(4-formylpiperazin-1-yl)deoxyclavulanat, 9-(4-Formylpiperazin-1-yl)deoxyclavulansäure, Benzyl-9-(4-phenylpiperazin-1-yl)deoxyclavulanat und 9-(4-Phenylpiperazin-1-yl)-deoxyclavulansäure.

7. Eine Verbindung, ausgewählt aus: Benzyl-9-(thiomorpholino)deoxyclavulanat, p-Nitrobenzyl-9-(thiomorpholino)deoxyclavulanat, 9-4-(Thiomorpholino)deoxyclavulansäure.

8. Eine Verbindung, ausgewählt aus: Benzyl-9-(morpholino)deoxyclavulanat, 9-(Morpholino)-deoxyclavulansäure, Benzyl-9-(2,6-dimethylmorpholino)deoxyclavulanat, 9-(2′,6′-Dimethylmorpholino)deoxyclavulansäure.

9. Eine Verbindung wie in irgendeinem der Ansprüche 1 bis 8 beansprucht, sofern sie in Zwitterionenform vorliegt.

10. Eine pharmazeutische Zusammensetzung, welche eine Verbindung wie in irgendeinem der Ansprüche 1 bis 9 beansprucht und einen pharmakologisch annehmbaren Trägerstoff enthält.

11. Eine Zusammensetzung wie in Anspruch 10 beansprucht, welche außerdem ein Penicillin oder Cephalosporin enthält.

12. Ein Verfahren zur Herstellung einer Verbindung wie in Anspruch 1 beansprucht oder eines pharmakologisch annehmbaren Salzes oder Esters derselben, welches Verfahren die Reaktion einer Verbindung der Formel V

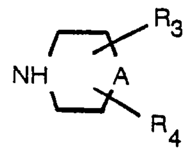

(V)

in welcher A, $R_3$ und $R_4$ wie in Anspruch 1 definiert sind, mit entweder einem Ester einer Verbindung der Formel (VI) oder einer Verbindung der Formel (VII) oder eines Salzes oder Esters derselben:

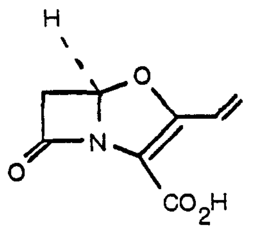

(VI)

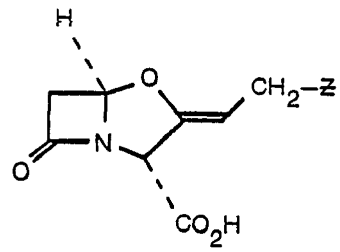

(VII)

in welcher Ƶ eine ersetzbare Gruppe bedeutet, umfaßt und anschließend, falls erforderlich, das Durchführen eines oder mehrerer der nachstehenden Verfahrensschritte:

i) Umwandeln eines Esters, falls vorhanden, in eine freie Säure oder ein Salz oder einen anderen Ester;

ii) Umwandeln der freien Säure oder eines Salzes in einen Ester;

iii) Bilden eines Säureadditionssalzes irgendeines solchen Esters.

13. Ein Verfahren wie in Anspruch 12 beansprucht, in welchem Ƶ eine Dichloracetoxygruppe ist.

14. Eine Verbindung wie in irgendeinem der Ansprüche 1 bis 9 beansprucht, zur Verwendung bei der Behandlung von bakteriellen Infektionen.

## Revendications

1. Composé de formule (II):

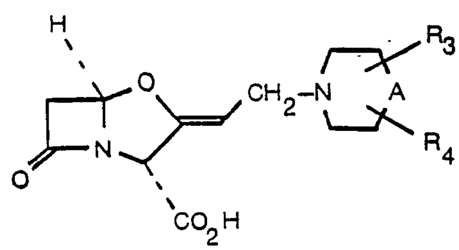

(II)

et sels pharmaceutiquement acceptables et esters de ce composé, formule dans laquelle A est un atome d'oxygène ou de soufre ou est un groupe $NR_5$ dans lequel $R_5$ est un atome d'hydrogène ou un groupe alcoyle en $C_{1-6}$, alcanoyle en $C_{1-6}$, alcoxy($C_{1-6}$)carbonyle, arylcarbonyle ou aralcoylcarbonyle, n'importe lequel de ces groupes étant facultativement substitué par un groupe alcoyle en $C_{1-3}$, alcoxy en $C_{1-3}$, hydroxy, alcanoyloxy en $C_{1-3}$, ou carboxy estérifié ou salifié; ou bien $R_5$ est un groupe de sous-formule (i):

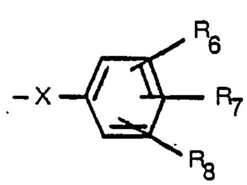

(i)

dans laquelle X est une liaison ou une fraction molaire —$CH_2$—; $R_6$ est de l'hydrogène, du fluor, du chlore, du brome ou un groupe alcoyle en $C_{1-3}$, alcoxy en $C_{1-3}$ ou hydroxy; $R_7$ est de l'hydrogène ou un alcoyle en $C_{1-3}$; et $R_8$ est de l'hydrogène ou un alcoyle en $C_{1-3}$; et $R_3$ et $R_4$ sont, indépendamment, de l'hydrogène ou un alcoyle en $C_{1-6}$.

2. Composé suivant la revendication 1, caractérisé en ce que $R_3$ et $R_4$ sont, indépendamment, des atomes d'hydrogène.

3. Composé suivant la revendication 1 ou 2, caractérisé en ce que $R_5$ est un groupe benzoyle, formyle, acétyle, propionyle, méthoxycarbonyle, éthoxycarbonyle, propoxycarbonyle, phénylacétyle ou hydroxyphénylacétyle.

4. Composé suivant la revendication 1 ou 2, caractérisé en ce que $R_5$ est un groupe phényle, fluorophényle, chlorophényle, bromophényle, méthylphényle ou méthoxyphényle.

5. Composé suivant la revendication 1 ou 2, caractérisé en ce que $R_5$ est un groupe benzyle, fluorobenzyle, chlorobenzyle, bromobenzyle, méthylbenzyle ou méthoxybenzyle.

6. Composé choisi parmi les suivants: 9-(4-benzylpipérazin-1-yl)désoxyclavulanate de benzyle, acide 9-(4-benzylpipérazin-1-yl)désoxyclavulanique, 9-(4-éthoxycarbonylpipérazin-1-yl)désoxyclavulanate de benzyle, acide 9-(4-éthoxycarbonylpipérazin-1-yl)désoxyclavulanique, 9-(4-formylpipérazin-1-yl)désoxyclavulanate de benzyle, acide 9-(4-formylpipérazin-1-yl)désoxyclavulanique, 9-(4-phénylpipérazin-1-yl)désoxyclavulanate de benzyle et acide 9-(4-phénylpipérazin-1-yl)désoxyclavulanique.

7. Composé choisi parmi les suivants: 9-(thiomorpholino)désoxyclavulanate de benzyle, 9-(thiomorpholino)désoxyclavulanate de p-nitrobenzyle, acide 9-(4-thiomorpholino)désoxyclavulanique.

8. Composé choisi parmi les suivants: 9-(morpholino)désoxyclavulanate de benzyle, acide 9-(morpholino)désoxyclavulanique, 9-(2,6-diméthylmorpholino)désoxyclavulanate de benzyle, acide 9-(2',6'-diméthylmorpholino)désoxyclavulanique.

9. Composé suivant l'une quelconque des revendications 1 à 8, lorsqu'il est sous la forme de zwittérion.

10. Composition pharmaceutique, caractérisée en ce qu'elle renferme un composé suivant l'une quelconque des revendications 1 à 9 et un véhicule ou excipient pharmaceutiquement acceptable pour celui-ci.

11. Composition suivant la revendication 10, caractérisé en ce qu'elle renferme également une pénicilline ou une céphalosporine.

12. Procédé pour la préparation d'un composé suivant la revendication 1 ou d'un sel pharmaceutiquement acceptable ou ester de celui-ci, ce procédé consistant à effectuer la réaction d'un composé de formule (V):

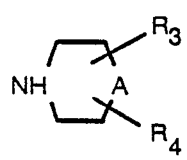

(V)

dans laquelle A, $R_3$ et $R_4$ ont la même signification que dans la revendication 1 avec, soit un ester d'un composé de formule (VI), soit un composé de formule (VII) ou un sel ou ester de celui-ci:

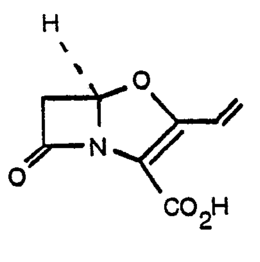

(VI)

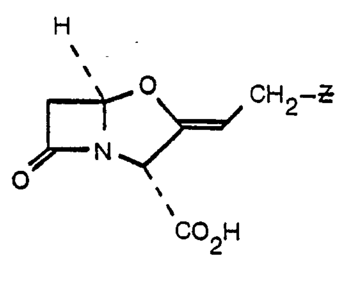

(VII)

dans lesquelles Ƶ est un groupe déplaçable, puis si nécessaire à effectuer un ou plusieurs des stades opératoires ci-aprés:

i) conversion d'un ester éventuellement présent en un acide libre ou bien en un sel ou un autre ester,
ii) conversion de l'acide libre ou du sel en un ester,
iii) formation d'un sel d'addition avec un acide de l'un quelconque de ces esters.

13. Procédé suivant la revendication 12, caractérisé en ce que Ƶ est un groupe dichloracétoxy.

14. Composé suivant l'une quelconque des revendications 1 à 9, destiné à être utilisé dans le traitement des infections bactériennes.